# EUROPEAN PATENT SPECIFICATION

(11) **EP 2 945 920 B1**
(45) Date of publication and mention of the grant of the patent: **05.08.2020**
(21) Application number: 14707848.9
(22) Date of filing: 16.01.2014
(51) Int. Cl.: C07C 1/12, C10G 2/00, B01J 37/03, B01J 23/00, B01J 23/745, B01J 23/78, B01J 27/22, B01J 37/08, B01J 37/16, B01J 35/00, B01J 35/10, B01J 37/00

(54) **A CATALYST AND A PROCESS FOR CATALYTIC CONVERSION OF CARBON DIOXIDE-CONTAINING GAS AND HYDROGEN STREAMS TO HYDROCARBONS**
KATALYSATOR UND VERFAHREN ZUR KATALYTISCHEN UMSETZUNG EINES KOHLENDIOXIDHALTIGEN GASES UND VON WASSERSTOFFSTRÖMEN ZU KOHLENWASSERSTOFFEN
CATALYSEUR ET PROCÉDÉ POUR LA CONVERSION CATALYTIQUE DE COURANTS D'HYDROGÈNE ET DE GAZ CONTENANT DU DIOXYDE DE CARBONE EN HYDROCARBURES

(30) Priority: 17.01.2013 US 201361753448 P
(43) Date of publication of application: 25.11.2015
(73) Proprietor: B.G. Negev Technologies and Applications Ltd., 8410501 Beer Sheva (IL)
(72) Inventor: LANDAU, Miron, 8483519 Beer-Sheva (IL); HERSKOWITZ, Mordechai, 8489332 Beer-Sheva (IL); VIDRUK, Roksana, 8479841 Beer-Sheva (IL)
(74) Representative: Vossius & Partner Patentanwälte Rechtsanwälte mbB
(86) International application number: PCT/IL2014/000006
(87) International publication number: WO 2014/111919

(56) References cited:
- B. D Roiter: "Phase Equilibria in the Spinel Region of the System FeO-Fe2O3-Al2O3", Journal of the American Ceramic Society, October 1964 (1964-10), pages 509-511, XP055117943, DOI: 10.1111/j.1151-2916.1964.tb13799.x Retrieved from the Internet: URL:http://onlinelibrary.wiley.com/store/1 0.1111/j.1151-2916.1964.tb13799.x/asset/j. 1151-2916.1964.tb13799.x.pdf?v=1&t=hv6k3ay u&s=f5e472eb3f24f5d653a5be17d26589e20f019d 7c
- J. W. HALLORAN ET AL: "Iron Diffusion in Iron-Aluminate Spinels", JOURNAL OF THE AMERICAN CERAMIC SOCIETY, vol. 63, no. 1-2, 1980, pages 58-65, XP055117775, ISSN: 0002-7820, DOI: 10.1111/j.1151-2916.1980.tb10650.x
- RESHETENKO T V ET AL: "Coprecipitated iron-containing catalysts (Fe-Al2O3, Fe-Co-Al2O3, Fe-Ni-Al2O3) for methane decomposition at moderate temperatures - I. Genesis of calcined and reduced catalysts", APPLIED CATALYSIS A: GENERAL, ELSEVIER SCIENCE, AMSTERDAM, NL, vol. 268, no. 1-2, 10 August 2004 (2004-08-10), pages 127-138, XP004512943, ISSN: 0926-860X, DOI: 10.1016/J.APCATA.2004.03.045
- M DRY ET AL: "The distribution of promoters in magnetite catalysts", JOURNAL OF CATALYSIS, vol. 7, no. 4, 1 April 1967 (1967-04-01), pages 352-358, XP055119210, ISSN: 0021-9517, DOI: 10.1016/0021-9517(67)90163-7
- A. M. RUBINSHTEIN ET AL: "Extensive study of the iron catalysts of ammonia synthesis. II. Structure and texture of twice promoted precipitated catalysts.", KINETIKA I KATALIZ, vol. 6, no. 2, April 1965 (1965-04), pages 285-293, XP002724739,
- THOMAS RIEDEL ET AL: "Fischer-Tropsch on Iron with H2/CO and H2/CO2 as Synthesis Gases: The Episodes of Formation of the Fischer-Tropsch Regime and Construction of the Catalyst", TOPICS IN CATALYSIS, KLUWER ACADEMIC PUBLISHERS-PLENUM PUBLISHERS, NE, vol. 26, no. 1-4, December 2003 (2003-12), pages 41-54, XP019292000, ISSN: 1572-9028 cited in the application
- KIM J S ET AL: "Performance of catalytic reactors for the hydrogenation of CO2 to hydrocarbons", CATALYSIS TODAY, ELSEVIER, NL, vol. 115, no. 1-4, 30 June 2006 (2006-06-30), pages 228-234, XP027975932, ISSN: 0920-5861 [retrieved on 2006-06-30]
- MIRON V. LANDAU ET AL: "Sustainable Production of Green Feed from Carbon Dioxide and Hydrogen", CHEMSUSCHEM, vol. 7, no. 3, 23 February 2014 (2014-02-23), pages 785-794, XP055117993, ISSN: 1864-5631, DOI: 10.1002/cssc.201301181
- NARAYANAN S ET AL: "ACETONE HYDROGENATION OVER CO-PRECIPITATED NI/AI2O3, CO/AI2O3 AND FE/AI2O3 CATALYSTS", JOURNAL OF THE CHEMICAL SOCIETY. FARADAY TRANSACTIONS, ROYAL SOCIETY OF CHEMISTRY, CAMBRIDGE, GB, vol. 94, no. 8, 21 April 1998 (1998-04-21) , pages 1123-1128, XP000767852, ISSN: 0956-5000, DOI: 10.1039/A708124C
- GIECKO G ET AL: "Fe2O3/Al2O3 catalysts for the N2O decomposition in the nitric acid industry", CATALYSIS TODAY, ELSEVIER, AMSTERDAM, NL, vol. 137, no. 2-4, 30 September 2008 (2008-09-30), pages 403-409, XP023438012, ISSN: 0920-5861, DOI: 10.1016/J.CATTOD.2008.02.008 [retrieved on 2008-03-25]

## Description

Land, air and marine transportation relies almost entirely on the availability of petroleum-derived liquid fuels. Given the limited availability of crude oil and the locations of the oil-producing countries, this dependence poses major security, economic, and environmental problems for the Western world.

Alternative routes for the production of synthetic liquid fuel from sources other than crude oil may be divided into three groups:
(i) conversion of coal and natural gas by a two-stage process, i.e., gasification to syngas (a mixture of CO and H₂), followed by Fischer-Tropsch (FT) synthesis;
(ii) conversion of biomass (cellulose, starch and lipids) by a wide variety of processes; and
(iii) conversion of carbon dioxide and water.

Among the three groups of conversion processes described above, the group using the most basic raw materials - carbon dioxide and water - is definitely the most attractive long-term alternative, with two main routes currently being explored with the aim of establishing the most viable process: artificial photosynthesis through photocatalytic and photoelectrochemical processes and water splitting combined with carbon dioxide hydrogenation. The development of the latter route is at a more advanced level, since both stages of the process have been successfully demonstrated at the bench scale.

Of the other two processes, those based on syngas are the most highly developed. Synthetic fuels have been produced for the past ninety years from coal and natural gas through a highly endothermic gasification process to syngas, followed by a highly exothermic FT process to produce paraffin mixtures, which are then further processed to a variety of final products.

Splitting of water generates hydrogen and oxygen. Oxygen can be combined with natural gas and steam to produce syngas that contains carbon monoxide, carbon dioxide and hydrogen by a process called autothermal reforming (L. Basini and L. Piovesan, "Reduction on Synthesis Gas Costs by Decrease of Steam/Carbon and Oxygen/Carbon Ratios in the Feedstock", Ind. Eng. Chem. Res. 1998, 37, 258-266; Kim Aasberg-Petersen, Thomas S. Christensen, Charlotte Stub Nielsen, Ib Dybkjær, "Recent developments in autothermal reforming and pre-reforming for synthesis gas production in GTL applications", Fuel Processing Technology 83 (2003) 253- 261). A similar process could be conducted with bio-gas instead of natural gas (McKenzie P. Kohn, Marco J. Castaldi, Robert J. Farrauto, "Auto-thermal and dry reforming of landfill gas over a Rh/y-Al2O3 monolith catalyst", Applied Catalysis B: Environmental 94 (2010) 125-133). Gasification is another option for converting biomass with oxygen to syngas (Richard C. Baliban, Josephine A. Elia and Christodoulos A. Floudas, "Biomass to liquid transportation fuels (BTL) systems: process synthesis and global optimization framework", Energy Environ. Sci., 2013, 6, 267-287). The syngas produced by all those methods is mixed with hydrogen produced by electrolysis and carbon dioxide from an external source to form a mixture of carbon dioxide, carbon monoxide and hydrogen that is reacted on tubular reactor packed with solid catalyst to generate an organic liquid and a gas that contain hydrocarbons and water containing oxygenates as a by-product.

While dealing extensively with the conversion to hydrocarbons of syngas (a mixture of CO and hydrogen that may contain some CO₂) via the Fischer-Tropsch synthesis, considerably lesser amount of work is reported in the art in respect of CO₂ hydrogenation. The feasibility of a process of carbon dioxide hydrogenation to form liquid fuels depends on the performance of the catalyst used to advance the reaction and on other process variables.

US 2,692,274 describes the catalytic reduction of carbon dioxide with hydrogen in the presence of iron oxide which contains 0.5% of copper and 0.8% K.

US 5,140,049 discloses a method for producing olefins from H₂ and CO₂ using potassium promoted catalysts of the Fe₃O₄/2%K and Fe_{2.85}CO_{0.15}O₄/2%K.

A series of catalysts based on iron and potassium supported on alumina, which optionally contain also copper, i.e., catalysts of the formulas Fe-K/Al₂O₃ and Fe-K-Cu/Al₂O₃ were reported in US 4,555,526, WO 97/05088 and WO 01/34538.

J.S.Hong et.al. [Applied Catalysis A, 218, 53-59, 2001] reported the synthesis of Fe-Al-Cu-K catalyst, its *in-situ* activation by means of hydrogen reduction at 450°C, followed by cooling down to 300°C, at which temperature the reaction was allowed to start in a continuous fixed bed reactor, into which CO₂:H₂ (1:3) streams were fed. After activation and stabilization at reaction conditions the catalyst contained two phases - iron oxide Fe₃O₄ and carbide Fe₅C₂. The best CO₂ conversion reported by these authors was around 40%.

Riedel et al. [Topics in Catalysis, Vol. 26, p. 41-54 (2003)] report the synthesis of Fe-Al-Cu-K catalyst by the quick addition of an aqueous NH₃ solution to a hot nitrate solution of iron, aluminum and copper. The precipitate was separated, washed and dried, and then impregnated with aqueous solution of potassium carbonate.

Non-stoichimetric, copper-containing spinel based on Fe-Al-O system was reported by Tanaka et. al. as being useful for exhaust gas treatment (JP 2011045840).

A. M. RUBINSHTEIN ET AL., "Extensive study of the iron catalysts of ammonia synthesis. II. Structure and texture of twice promoted precipitated catalysts." in KINETIKA I KATALIZ, vol. 6, no. 2, April 1965 (1965-04), pages 285-293 discloses iron catalysts for ammonia synthesis.

It has now been found that liquid fuels can be prepared with high productivity by a reaction of carbon dioxide with hydrogen on solid porous catalyst based on iron. The catalyst operative in the present invention comprises iron-containing spinel phase (before activation). In its most general form, spinel phase indicates a mixed oxide having the formula A²⁺B³⁺₂O₄. According to the present invention, the divalent ion A²⁺ is Fe²⁺ and is essentially free of Cu²⁺, whereas the trivalent ion B³⁺ is Fe³⁺ in combination with Al³⁺. Thus, the spinel component of the catalyst is represented by formula 1: Fe²⁺(Fe³⁺_{yAl}³⁺_{1-y})₂O₄ (spinel of Formula 1) wherein y is in the range from 0.25 to 0.75; (Cu²⁺ₓFe²⁺₁₋ₓ)(Fe³⁺_{y}Al³⁺_{1-y})₂O₄ (spinel of Formula 2; not according to the presently claimed invention) wherein x is in the range from 0.0 to 0.05, i.e., 0.0<×≤0.05 and y is in the range from 0.05 to 0.95.

According to the notation used herein, a catalyst which consists of hematite and spinel phases together is represented by the formulas:

α[Fe₂O₃]β[Fe²⁺(Fe³⁺_{y}Al³⁺_{1-y})₂O_{4]} Formula I

α[Fe₂O₃]β[(Cu²⁺ₓFe²⁺₁₋ₓ)(Fe³⁺_{y}Al³⁺_{1-y})₂O₄] Formula II

wherein α is <5 wt% and β >95wt%, correspondingly, and x and y are as previously defined.>

According to the presently claimed invention, however, the catalyst according to the invention does not contain hematite phase and is copper-free. Thus, the catalyst according to the presently claimed invention consists of potassium promoted spinel of Formula 1: K/Fe²⁺(Fe³⁺_{y}Al³⁺_{1-y})₂O₄, wherein y is in the range from from 0.25 to 0.75, more preferably from 0.3 to 0.7, and specifically from 0.4 to 0.6, with the weight ratio Fe:Al:K preferably varying from 100:20:3 to 100:30:10. The formula K/Fe²⁺(Fe³⁺yAl³⁺1_{-y})₂O₄ indicates that the potassium is applied onto the surface of the spinel of Formula 1.

The catalyst of the invention is prepared by a process comprising dissolving in an aqueous solution at least one ferric compound and at least one aluminum compound, and gradually adjusting the pH of the metal-containing solution, to about 7.0 - 8.5, by means of an addition of ammonium hydroxide, whereby co-precipitation of the metals in the form of their hydroxides occurs, separating the solid formed from the liquid phase, and subjecting same to drying and calcination (for dehydration and formation of the spinel phase).

Suitable starting materials for use in the preparation of the catalyst are water-soluble ferric and aluminum salts, such as the nitrate salts. The metal nitrates exist in hydrated forms: Fe(NO₃)_{3̇}·9H₂O and Al(NO₃)₃·9H₂O, which are especially preferred. The preferred concentrations of the ferric and aluminum salts in the aqueous solution are from 1900 to 2100 and 900 to 1100 gram/L, respectively, with the relative amounts of the salts being adjusted to form the desired composition of the catalyst. The water-soluble metal salts, e.g., the nitrates, can be added to the aqueous solution in a solid form in any desired order, or can be pre-dissolved in separate solutions, which are subsequently combined together. According to the presently claimed process, the weight ratio Al:Fe in the solution is not less than 20:100, e.g., from 20:100 to 30:100.

The experimental results reported below indicate that the best catalysts of the invention are the copper-free pure spinel of Formula 1 promoted with potassium. However (not according to the presently claimed invention), the presence of small amounts of copper, i.e., the catalysts (Cu²⁺ₓFe²⁺₁₋ₓ)(Fe³⁺_{y}Al³⁺_{1-y})₂O₄ (spinel of Formula 2) can still be used, provided that x is less than 0.05. In the event that the catalysts of Formula 2 are contemplated, cupric salt, e.g., Cu(NO₃)₂·3H₂O is added to the solution described above.

The adjustment of the pH of the aqueous solution to the range from 7.0-8.5, especially from 7.5 to 8.0, in order to affect the co-precipitation of the metal hydroxides, is accomplished by means of gradual addition of an aqueous solution of ammonium hydroxide, which solution is preferably applied in a dilute form, with concentration of not more than 5% by weight. The gradual addition preferably lasts not less 10 minutes, e.g., not less than 20, 30 or 60 minutes, and may be accomplished by portionwise addition or feeding a stream of ammonium hydroxide solution at a suitably slow flow rate. During pH adjustment, the solution of the metals and the slowly added NH₄OH solution are preferably held at a temperature below 30°C, e.g., at room temperature. The experimental results reported below indicate that a sharp increase of the pH of the aqueous metal solution (e.g., due to a rapid addition of a concentrated alkaline solution) may unfavorably alter the composition of the catalyst, ultimately causing the formation of the hematite phase following the calcination stage. Working with Al:Fe weight ratio as set out above and carefully basifying the solution afford, following the calcination, the hematite-free spinel of Formula 1.

The precipitate formed is separated from the mother liquor, e.g., by filtration or any other solid/liquid separation technique, and is preferably washed with water and dried at temperature in the range of 100-140°C for period of at least 24 h. Before the calcination step, the dried material is preferably treated with an aqueous solution of a potassium salt, e.g., the solid is impregnated with a solution of potassium carbonate, nitrate or acetate until incipient wetness is observed, followed by drying. The impregnation-drying cycle may be repeated several times, in order to load the potassium solution onto the surface of the solid. The presence of potassium renders the surface of the catalyst slightly alkaline, and hence increases the affinity of the catalyst towards carbon dioxide, which is an acidic gas. In general, the weight ratio Fe:K is in the range from 100:3 to 100:10.

The solid is then subjected to drying in air at a temperature in the range of 100-140°C for period of at least 24 hours followed by calcination in air at a temperature in the range from 400 to 500°C for period of at least 6 h.

The catalyst obtained exhibits surface area of not less than 80 m²/gram, e.g., between 80 and 180 m²/gram; pore volume of not less than 0.1 cm³/gram, e.g. between 0.1 and 0.5 cm³/gram, and average pore diameter between 3.0 and 7.0 nm. Elemental analysis by means of energy dispersive X-ray spectroscopy of the preferred catalyst of Formula 1 indicates that the weight ratio Fe:Al is in the range from 100:20 to 100:30. X-Ray Diffraction (XRD) indicates that the preferred catalyst of Formula 1 has average crystal size of 1.5 to 3 nm.

The experimental results reported below indicate that the use of a pure copper-free Al-substituted spinel phase, i.e., the compound of Formula 1, in a nano-sized form, results in an increase in the CO₂ conversion and also in a better reaction selectivity towards the heavier, liquid hydrocarbons (designated C₆₊). The spinel component, after activation and self-organization at CO₂ hydrogenation conditions, is highly active and capable of supporting the conversion of carbon dioxide at an appreciable extent to higher hydrocarbons C₅₊. When the hydrogenation of CO₂ is carried out in the presence of a spinel catalyst containing a hematite component, its activity is lower due to the formation of a large amount of carbon deposits blocking the active phases. This is a result of oxidation of Fe-carbide phases formed during hematite carburization at the activation step by reactions with CO₂ and water formed in the process:

3 Fe₅C₂ + 3CO₂ = C + 2CO +5 Fe₃O₄

3FeₛC₂ + 20H₂O = 5Fe₃O₄ + 20H₂ + 6C

Conversely, the hematite phase alone, which transforms at the activation step to carbides Fe₅C₂ and Fe₇C₃, is capable of producing the desired high olefins/paraffins, but is not sufficiently active to permit acceptable CO₂ conversion. However, a catalyst which consists of pure K-promoted spinel phase of Formula 1, particularly when subjected to specific activation conditions and employed in a continuous process running through a set of successively arranged reactors, namely, a cascade of reactors, demonstrates an excellent activity. The high activity is amounting to depressing of the formation of carbon deposits and stabilization of active carbide phases in a modified spinel matrix. This modified spinel matrix has less Fe content reduced at activation and *in situ* self-organization of the parent spinel phase due to partial removal of Fe-ions forming carbide phases Fe₅C₂ and Fe₇C₃. It appears that the presence of copper (not according to the presently claimed invention) [i.e., in the spinel of Formula 2:

(Cu²⁺ₓFe²⁺₁₋ₓ)(Fe³⁺_{y}Al³⁺_{1-y})₂O₄]

facilitates reduction of iron ions during activation and at CO₂ hydrogenation conditions. We have found that copper is redundant when the activation is accomplished by means of carburization.

It should be noted that the catalyst of the invention can be processed to form pellets. To this end, the powdery material collected following the precipitation and separation of the spinel of Formula 1 Fe²⁺(Fe³⁺_{y}Al³⁺_{1-y})₂O₄ from its mother liquor is combined with a binder such as aluminum hydroxide (bohemite) powder and water to form a workable slurry. For example, the weight ratio Fe²⁺(Fe³⁺_{y}Al³⁺_{1-y})₂O₄: alumina: water is from 1:0.14-0.71:0.43-0.86 . The mixture is extruded and the so-formed extrudates are loaded with potassium, e.g., by means of the aforementioned impregnation with potassium carbonate solution, followed by drying and calcination.

Another way to form pellets from the spinel of Formula 1 Fe²⁺(Fe³⁺_{y}Al³⁺_{1-y})₂O₄ is by means of pressurizing the calcined powder after insertion of potassium using special form that yield cylindrical or empty cylindrical pellets.

In use, the catalyst of the invention is loaded into a suitable reactor, e.g., a continuous fixed bed reactor, or into a plurality of consecutively arranged reactors, as described in more detail below. The catalyst may be applied either in a powder or granular form. For example, the particle size of the catalyst used may be in the range from 100 *µ*m to 3 mm. Thus, the powdery catalyst collected following the calcination step may be granulated, milled and then passed through a suitable sieve in order to collect the desired population of particles. The catalyst may also be applied in the form of pellets. The thickness of the catalyst layer packed in the reactor may vary within the range of 7 mm to 3 m provided the particle size is selected so as to avoid excessive pressure drop.

In order to be operative in the carbon dioxide hydrogenation process, the catalyst needs first to undergo activation. The activation of the catalyst is carried out *in-situ* by carburization.

Reduction (not according to the claimed process) involves the flow of hydrogen stream through the reactor in which the catalyst is placed at a temperature of not less than 400°C. The flow rate of the hydrogen stream is not less than 100 cm³/min per gram of catalyst. The reduction is continued at atmospheric pressure for not less than 3 hours. After that the temperature is adjusted in hydrogen flow according to the process conditions and a CO₂/H₂ mixture is fed to the reactor.

Carburization involves the exposure of the catalyst to a carbon-containing atmosphere. To this end, streams of carbon monoxide, hydrogen and an inert gas carrier are caused to flow through the reactor in which the catalyst is placed at a temperature of not less than 300°C. The flow rates of the three gaseous components (CO, H₂ and inert gas (N₂, He, Ar)) are at least 30:30:150 cm³/min per one gram of the catalyst, respectively. The carburization is continued at atmospheric pressure for not less than 3 hours.

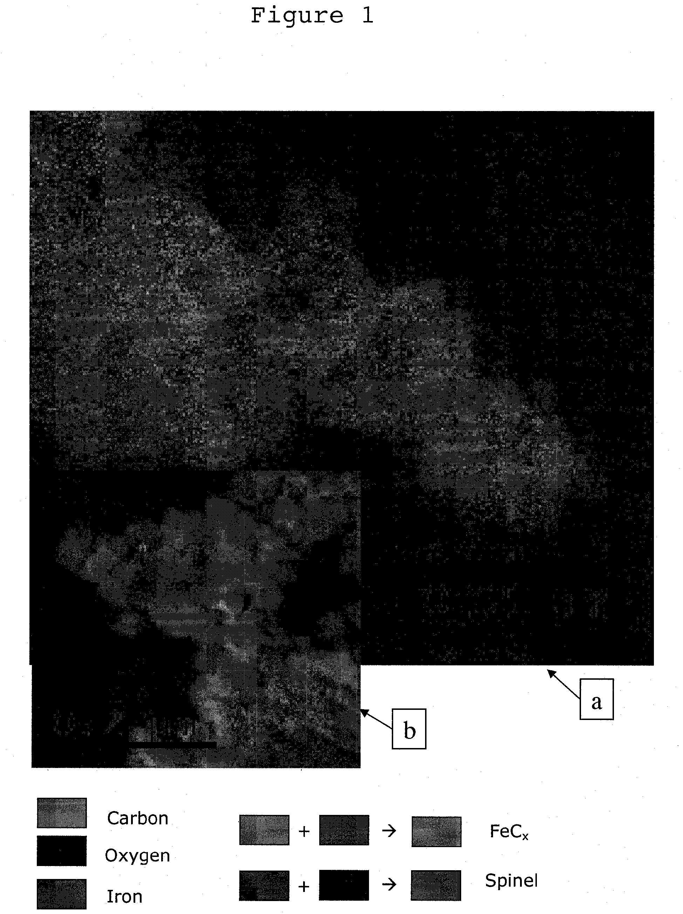

The experimental results reported below indicate that the mode of catalyst activation is of significance. The reduction leads to the transformation of the iron ions in spinel phase into the metallic iron that is subsequently converted to a mixture of Fe₃O₄ inactive in Fischer-Tropsh reaction and catalytically active FeCₓ carbide phases. Activation by means of carburization is according to the claimed invention since it converts iron ions existing in the spinel phase in the starting catalyst directly to iron carbide phases of higher dispersion (particles of 20-35 nm) leaving the Fe-impoverished spinel phase as aggregates of nanoparticles of higher dispersion (8-10 nm) randomly mixed with iron carbide nanoparticles (Figure 1b). After hydrogen reduction and stabilization at reaction conditions the nanoparticles of carbide and spinel phases are larger (50 - 100 nm) and separated from each other by carbon that decreased the contact interface between carbide and spinel phases (Figure 1a).

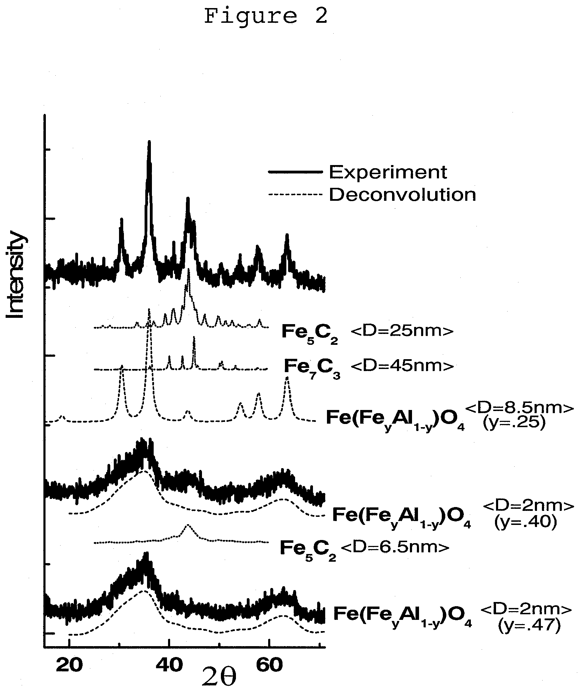

Typical X-ray diffraction patterns of the fresh, activated (carburization) and stabilized at CO₂/H₂ hydrogenation conditions catalyst of the present invention are presented in Figure 2. The fresh catalyst consists of spinel phase of Formula 1 of high dispersion with crystal size of 1.5 to 3 nm, e.g., around 2 nm. After activation by carburization and stabilization in CO₂/H₂ hydrogenation at 300°C for 100-150 h, e.g., 120 h, the spinel phase is converted to Fe-carbide phases with crystal size of 25 and 45 nm while the crystal size of the spinel phase increases to 8.5 nm with decreasing the y value in its formula Fe(Fe_{y}Al_{1-y})₂O₄ from 0.47 to 0.25. It means that part of the iron in the spinel phase during activation-stabilization is converted to carbides. It is believed that the activity of the catalysts of the invention resides in the combination of the catalytic action of two phases - spinel catalyzing presumably the reverse water gas shift reaction producing CO, and iron carbides catalyzing both reverse water gas shift and Fischer-Tropsch reaction that converts CO/H₂ to hydrocarbons.

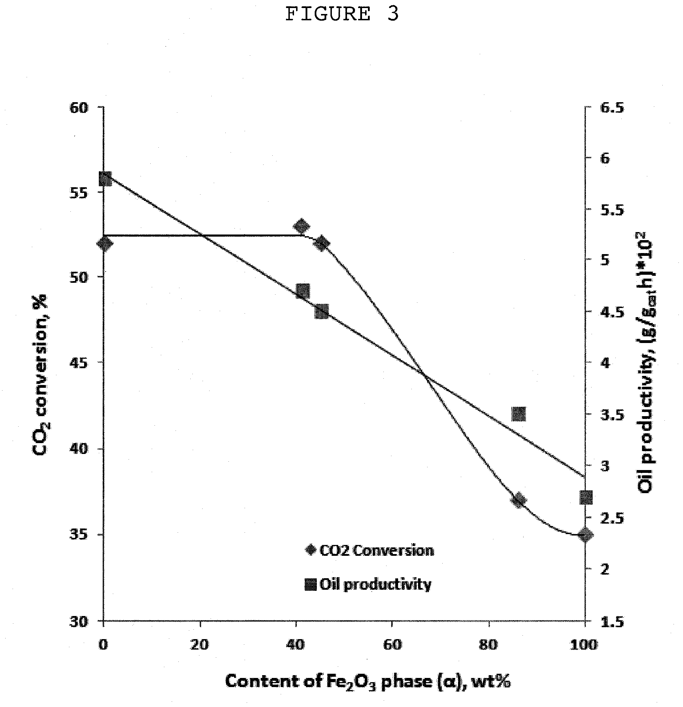

The effect of hematite phase content that may exist in the catalyst together with the substituted spinel phase at different amounts depending on the synthesis conditions (not according to the presently claimed invention) is clearly illustrated in Figure 3, which compares the CO₂ conversions and liquid hydrocarbon oil productivities measured in testing examples of the present invention and comparative examples at standard conditions. As shown in Figure 3, the content of hematite phase in copper-free catalysts was in the range from 0 (pure spinel) to 100% (pure Fe-oxides). The catalyst containing pure spinel without Fe-oxide phase (according to the presently claimed invention) demonstrated the most efficient combination of catalytic activity and selectivity to C₆₊ hydrocarbons reflected by highest productivity of C₆₊ hydrocarbons. The pure Fe-oxide phase without Al-, mixed Fe-Al-O-spinel - Fe₂O₃ materials or Cu-Al containing spinel promoted with potassium (not according to the presently claimed invention) after activation display much lower activity in CO₂ hydrogenation to hydrocarbons than the catalyst consisting of pure Fe-Al-O spinel phase.

Accordingly, another aspect of the invention is a process for the preparation of hydrocarbons according to claim 10 comprising the hydrogenation of carbon dioxide-containing gas in the presence of iron-containing catalyst devoid of hematite phase, said catalyst being copper-free, wherein the catalyst is activated *in-situ* by means of carburization, said carburization comprises treating said catalyst with a mixture of H₂ and CO at elevated temperature. Following carburization, the content of the carbide phases in the catalyst is less than 15 wt%, preferably from 5 to 10 wt%, relative to the total weight of the catalyst.

Following the *in-situ* activation of the catalyst, the carbon dioxide hydrogenation reaction is allowed to start. Carbon dioxide-containing gaseous stream and hydrogen stream are continuously fed to the reactor at H₂/CO₂ of not less than 2 and weight hourly space velocity (WHSV) not less than 0.1 h⁻¹, preferably not less than 0.5 and more preferably not less than 1. The reaction is carried out at a temperature in the range from 250 to 360°C at pressure of not less than 5 atmospheres, e.g., from 10 to 30.

It should be noted that the carbon dioxide-containing gas stream used in the present invention may be neat carbon dioxide and also any gas mixture which contains carbon dioxide, for example, a mixture of carbon dioxide and carbon monoxide, with the molar fraction of carbon dioxide being not less than 0.25, more specifically not less than 0.5.

As already noted above, it has been found that it is advantageous to conduct the catalytic hydrogenation of carbon dioxide-containing gaseous stream via a cascade process, namely, by using a plurality of fixed bed reactors arranged is series. The chief advantage offered by the cascade process of the invention resides in that it allows the introduction of intermediary step of water removal between each pair of serially-connected reactors. It has been found that water, which is a co-product of the reaction, has a double negative effect on the catalysts performance. First, the partial pressure of the water, which increases along the catalyst bed from inlet to outlet, inhibits the reverse water-gas shift reaction: CO₂ + H₂ → CO+H₂O, thus it limits the CO₂ conversion. Second, at high partial pressure of water the active carbide Fe₅C₂ phase is oxidized to iron oxide that deactivates the catalyst. As a result, CO₂ conversion in one reactor reaches a limited value, well below 100%. (the experimental results reported below indicate that conversion measured at 300°C, WHSV <1 h⁻¹ and total pressure of 10 atm asymptotically approaches - 60%).

Accordingly, one aspect of the invention relates to a cascade process for hydrogenation of carbon dioxide-containing gaseous stream employing a plurality of serially arranged reactors as defined in present claim 11, comprising:
(i) feeding carbon dioxide-containing gas stream and hydrogen stream into at least the first of said serially arranged reactors which are loaded with iron-containing catalyst, said catalyst being copper-free, wherein said catalyst was brought to an active form by means of carburization;
(ii) discharging a gaseous reaction stream from at least one of said serially arranged reactors and cooling same, to form a liquid-gas mixture, wherein the liquid component of said mixture comprises condensable reaction products consisting of water and organic liquids, and the gas component of said mixture comprises reactants and non-condensable reaction products;
(iii) removing said gaseous component from said liquid component;
(iv) separating said liquid component into an organic and aqueous phases, and collecting at least said organic phase;
(iv) directing the removed gaseous component into the successive reactor in said serially arranged reactors.

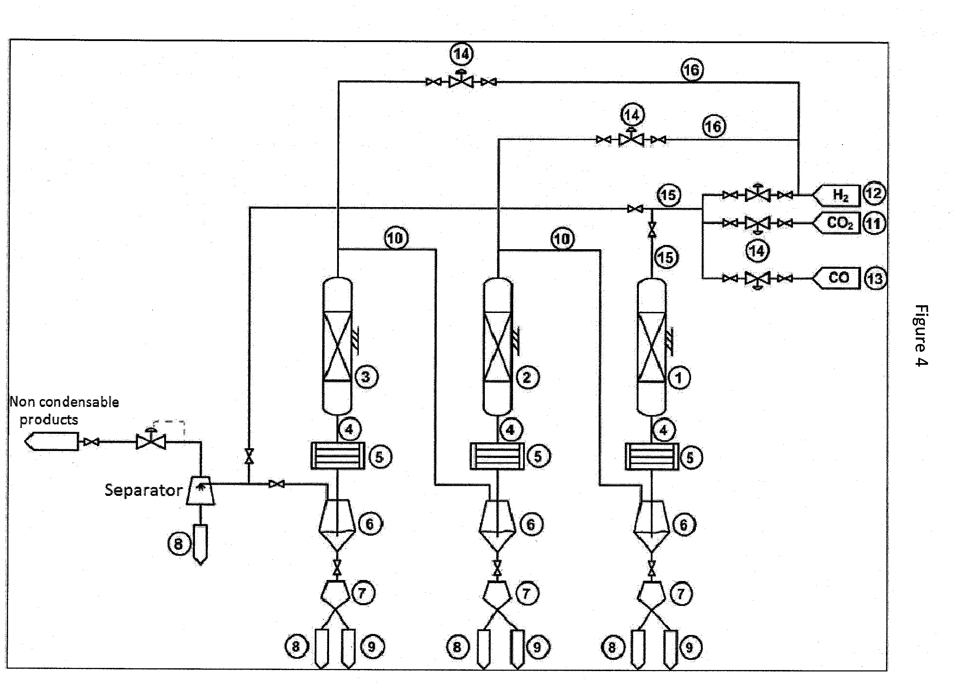

A suitable apparatus for carrying out the process of the invention is schematically illustrated in Figure 4, in which the cascade configuration consists of three serially arranged reactors.

The serially-positioned reactors are designated by numerals (1), (2) and (3). Each reactor is provided with a suitable amount of a catalyst loaded therein. The amount of catalyst in each reactor is adjusted to improve conversion and productivity. Alternatively, the total amount of the catalyst used in the process may be equally divided between the set of reactors.

Each reactor is provided with a discharge line (4), with a cooler (5), a gas-liquid separator (6) and a liquid-liquid separator (7) positioned successively along said discharge line. Each liquid-liquid separator (7) is connected to two tanks, (8) and (9), for collecting organic and aqueous phases, respectively. The gas-liquid separator is connected by means of a feed line (10) into the consecutive reactor.

Carbon dioxide, hydrogen and optionally carbon monoxide required for the process are supplied through feed lines (11), (12) and (13), respectively. Hydrogen may be produced by the electrolysis of water in an electrolysis unit (not shown) and carbon dioxide may be recovered from industrial processes such as the combustion of fossil fuels in power generating plants, cement or steel plants. Another useful source of the reactans required for the process is carbon dioxide-rich syngas, i.e., a gas mixture comprising CO₂, H₂ and CO where the concentration of CO₂ is greater than the normal concentration in conventional syngas, i.e., the molar fraction of carbon dioxide in the syngas is not less than 0.25 and preferably not less than 0.5.

In the specific embodiments shown in Figure 4, separate CO₂, H₂ and CO feeds are shown, equipped with flow controllers (14), and the gases are combined in a feed line (15) and introduced into the first reactor. While the description that follows relates to the embodiment illustrated in Figure 4, it should be noted that a combined gaseous stream, e.g., carbon dioxide-rich syngas, may be utilized.

In operation, carbon dioxide (11) and hydrogen (12), and optionally carbon monoxide (13) streams are fed to the first of said serially arranged reactors, with their relative amounts being adjusted by means of flow controllers (14). As shown in Figure 4, hydrogen may be directly fed also to each of the successive reactors by means of feed lines (15, 16) equipped with flow controllers (14), so as to maintain an optimal ratio between the gaseous reactants. For example, CO₂, H₂ and CO may be fed at molar ratios 1:5:1.

The temperature and pressure maintained within each of the serially-placed reactors are as set forth above, and the preferred WHSV, when using the copper-free catalyst of the invention, i.e., of Formula 1, is not less than 0.5.

The gaseous mixture produced at each reactor is discharged (4), and subjected to cooling and condensation (5) whereby a liquid-gas mixture is obtained. This mixture is separated into its liquid and gaseous components in a gas-liquid separator (6).

The gaseous, non-condensable component, which consists of non-reacted CO₂, H₂ and CO with light organic compounds is removed from the liquid component and fed directly to the next reactor via line (10). The liquid component is fed to a liquid-liquid separator (7), where it is separated into organic and aqueous phases, which are collected in two distinct tanks. The organic product (8) (containing mainly alkanes and alkenes with 6-20 carbon atoms in their molecules) is further processed to produce high-quality liquid fuels by methods known in the art. As shown in Figure 4, a further separation may be conducted after the third stage, to separate liquids from non-condensable products.

Notably, the cascade configuration illustrated in Figure 4 removes the limitations on CO₂ conversion rendered by high water partial pressure in the downstream parts of the catalysts layer in one fixed-bed reactor and allows reaching CO₂ conversions of > 75% and up to > 90%.

Implementation of the reactors cascade arrangement according to the present invention, with water removal between each pair of succssive reactors, allows to get higher CO₂ conversions and oil productivities in comparison to operting with the same catalyst but in a single reactor. This improvement is obtained irrespective of the type of catalyst used (either pure Fe-oxide or spinel or mixed catalysts) and composition. Implementation of cascade reactors configuration according to the present invention removes the limitation of - 60% CO₂ conversion that appears to be the upper limit when using Fe-based catalysts in a single reactor. This allows reaching >90% CO₂ conversion at WHSV > 1 h⁻¹.

The present invention permits obtaining the liquid hydrocarbon oil as an excellent feedstock for production of transportation fuels: gasoline, jet fuel and diesel fuel according to well established technologies [A.de Klerk, Energy Environ. Sci., 2011, 4, 1177]. The oil productivity at similar total WHSV obtained in three reactors in series increases by about factor of 3 and reaches 170-520 gram of oil per kilogram of catalyst per hour. This corresponds to the productivity of about 0.5 million ton oil /year of the unit with 100 ton catalysts loading. The catalysts according to the present invention tested in a cascade reactors arrangement demonstrated excellent stability in 500 hour on stream runs.

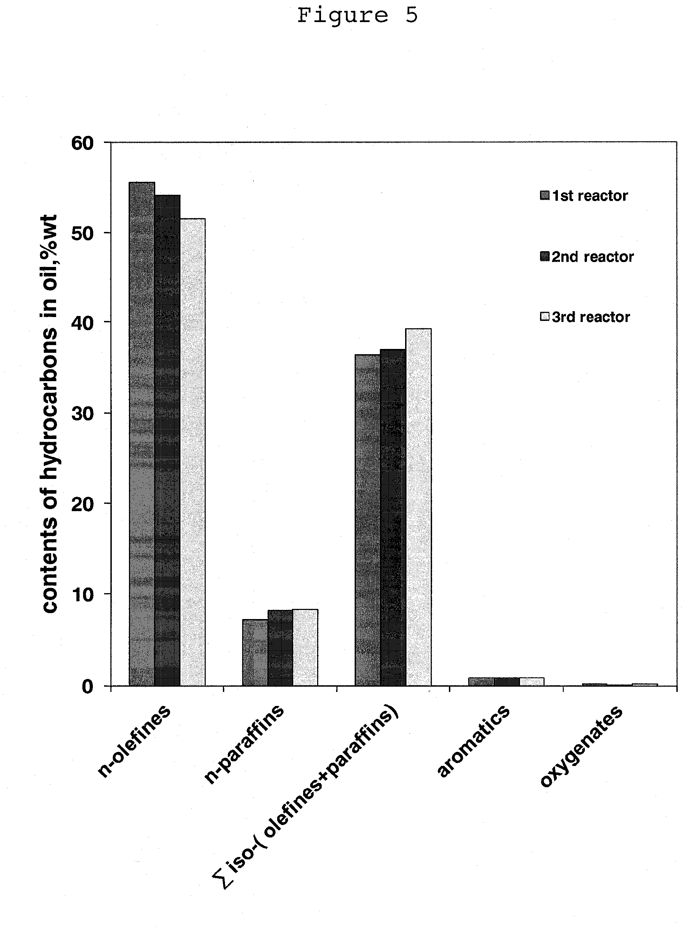

A typical chromatogram of the liquid oil produced by the hydrogenation process of the invention indicates that the oil consists of hydrocarbons with 6 to 27 carbon atoms where distribution maximum is centered at 10-12 carbon atoms. As shown in the bar diagram of Figure 5, the hydrocarbon compositions of the liquid products formed in each of the three consecutive reactors are comparable (in each set of adjacent bars, the left, middle and right bars correspond to the first, second and third reactors placed in series) The oil contains about 60 wt% of n-olefins and n-paraffins at weight ratio of 5:1, about 1 wt% aromatics, 0.1-0.2 wt% oxygenates and the rest - iso- olefins and iso-paraffins at weight ratio of 4:1. The water fraction separated from oil contains 2-5 wt% of total organic carbon in form of light oxygenates.

Distillation patterns demonstrate that the organic liquid obtained in CO₂ hydrogenation according to the present invention can be separated by distillation to gasoline, jet and/or diesel fuels fractions according to technical needs. These fractions may be further upgraded to fit the fuels specification requirements by means of methods known in the art, e.g., as it is done for hydrocarbon products obtained in a regular CO/H₂ Fischer-Tropsch process [A.de Klerk, *supra*]*.*

### In the drawings:

Figure 1 shows the multicomponent elements distribution maps of K/Fe-Al-O-spinel catalyst obtained by HRTEM/EELS: (1) - after hydrogen reduction and stabilization in CO₂ hydrogenation at 300°C for 120 h; (2) - after carburization and stabilization in CO₂ hydrogenation at 300°C for 120 h.
Figure 2 shows XRD patterns of K/Fe-Al-O-spinel catalyst: (1) - fresh; (2) - after activation by carburization; (3) after stabilization in CO₂ hydrogenation at 300°C for 120 h.
Figure 3 demonstrates the effect of the content of Fe₂O₃ (Hematite) phase in the mixed Fe₂O₃-Fe-Al-O-spinel catalyst on the catalysts performance in CO₂ hydrogenation. Testing conditions: T = 300°C, Pₜₒₜₐₗ = 10 atm, H₂/CO₂ = 4.
Figure 4 illustrates a scheme of an apparatus based on a cascade reactors system useful for CO₂ hydrogenation. (1), (2) and (3) serially-positioned reactors; (4) discharge line; (5) cooler; (6) gas-liquid separator; (7) liquid-liquid separator; (8) organic collector; (9) aqueous phases collector; (10) feed line into the consecutive reactor; (11), (12) and (13) carbon dioxide, hydrogen and carbon monoxide feed lines, respectively; (14) flow controllers; (15), (16) gas feed lines.
Figure 5 is a bar diagram illustrating the contents of paraffins, olefins, aromatics and oxygenates in the hydrocarbon oil obtained after one, two and three reactors in series.

### Examples

### Methods

X₋Ray Diffraction (XRD) The X-ray diffraction (XRD) patterns were obtained with a Phillips 1050/70 powder diffractometer fitted with a graphite monochromator, at 40 kV and 28 mA. Software developed by Crystal Logic was used. The data were collected in a range of 2θ values between 5° and 80° with a step size of 0.05°. Phase identification was performed by using BEDE ZDS computer search/match program coupled with the ICDD (International Center for Diffraction Data) Powder Diffraction File database (2006). The average crystal size of Fe₃O₄, Fe²⁺(Fe³⁺_{y}Al³⁺_{1-y})₂O₄ spinel, Fe₂O₃, Fe₅C₂ and Fe₇C₃ phases was determined from the Scherrer equation h = Kλ/[(B²- β²)^{0.5} cos(2θ/2)], where K = 1.000 is the shape factor, λ = 0.154 nm, β is the instrumental broadening correction, and B is the reflection broadening at corresponding 2θ. The average crystal size was obtained by averaging of the data calculated for reflections (110), (200); (220), (311); (220), (311), (440); (020), (112); (102), (211) and (110), (200) of the XRD patterns corresponding to the Hematite Fe₂O₃ phase (ICDD Card 86-550), Magnetite Fe₃O₄ (ICDD Card 19-629), Cu-Al-Fe-O spinels (ICDD Cards 34-192, 77-10), Hägg carbide Fe₅C₂ (ICDD Card 36-1248), carbide Fe₇C₃(ICDD Card 17-333) and metallic α-Fe (ICDD Card 6-696) phases, respectively. The relative content of Fe-oxides, carbide phases and amorphous carbon phase represented in X-ray diffractograms by a wide reflection centered at 2*θ* = 22° was obtained by Rietveld refinement of the XRD profile by using the DBWS-9807 program.

The composition of spinel phases nanoparticles in prepared catalytic materials was derived from XRD data - by fitting the measured parameters of the unit cell of cubic Fe₂O₃-phase to the contents of Cu²⁺ and Al³⁺ ions at tetrahedral and octahedral positions, respectively, in the cubic framework of spinel phase with space group Fd3m. The parameters of unit cells of spinel phases were calculated based on the positions of reflections detected in X-ray diffractograms around 2θ =30° (220), 35.5° (311) and 63° (440).

### Surface area and pore volume measurements

Surface area and pore volume were derived from N₂ adsorption-desorption isotherms using conventional BET and BJH methods (Barrett-Joyner-Halenda method, Journal of American Chemical Society, 73, 373, 1951). The samples were degassed under vacuum at 250-70°C, depending on their thermal stability. Isotherms were measured at liquid nitrogen temperature with a NOVA-2000 Quantachrome, Version 7.02 instrument.

### Energy dispersive X-ray spectroscopy (EDAX)

The total elemental composition of catalysts was measured by EDAX method using Quanta-200, SEM-EDAX, FEI Co. instrument. The contents of Cu²⁺ and Al³⁺ ions in spinel nanoparticles were confirmed by the data of local EDAX method recorded with the electronic spot diameter of 5 nm with the field emission analytical transmission electron microscope (JEOL JEM-2100F) operated at accelerating voltage of 200 kV. The nanoparticles of spinel phases were identified by recording the multicomponent distribution maps for nanoparticles observed at HRTEM images.

### High resolution TEM / electron energy loss spectroscopy (HRTEM/EELS)

The transmission electron microscope (JEOL JEM-2100F) used for materials analysis was equipped with a Gatan imaging filter (GIF Quantum) / EELS system. Regular and energy-filtered images were taken from the same samples point for Fe, O and C elements at L-edge 708 eV; K-edge 532 eV and K-edge 284 eV, respectively. The obtained elemental distributions maps were further combined in one RGB image marking Fe by red, C by green and O by blue colors. This yielded multicomponent elements distribution maps for nanoparticles observed in regular HRTEM images. Gatan Digital Micrograph software was used for EFTEM imaging, recording and processing of EELS data. The samples for HRTEM were prepared by depositing a drop of ethanol suspension of the solid catalyst on a silica-coated copper grid that formed a contrast oxide background for carbon-rich particles.

### Example 1 (comparative)

### Preparation of a catalyst consisting of hematite phase [Fe₂O₃] and spinel phase of the formula [(Cu²⁺ₓFe²⁺₁₋ₓ)(Fe³⁺_{y}Al³⁺_{1-y})₂O₄; (x=0.54, y=0.65)]

The catalyst was prepared by co-precipitation from the aqueous solution of Fe, Cu and Al nitrates by addition of aqueous ammonium hydroxide solution. 21.0 gram of Al(NO₃)₃·9H₂0, 72.3 gram of Fe(NO₃)₃·9H₂O and 6.8 gram of Cu(NO₃)₂·3H₂O salts were dissolved in 20 , 20 and 4 cm³ of distilled water, respectively. All three solutions were then mixed together, and 500 cm³ of 3% NH₄OH solution were gradually added to this mixed salts solution at room temperature under stirring during a period of 10 min. The pH of solution after the addition of NH₄OH was 6.8. A solid precipitated from the solution. The solid was separated by filtration and washed on the filter with 500 cm³ of distilled water. Then the solid was dried in air at 110°C for period of 24 h. The obtained material was impregnated by incipient wetness with aqueous solution containing 1.04 gram K₂CO₃. The impregnated material was dried in air at 110°C for period of 48 h and calcined in air at 450°C for period of 6 h (temperature increasing rate 5°C/min). The material has the following weight ratio of metal components (EDAX): Fe:Al:K:Cu = 100:15:6:5, surface area 100 m²/g, pore volume 0.17 cm³/g and average pore diameter 4.3 nm. The material contained two phases (XRD): 30 wt% of hematite Fe₂O₃ and 70 wt% of phase with spinel structure which composition reflects partial isomorphous substitution of Fe²⁺ ions with Cu²⁺, and Fe³⁺ ions with Al³⁺ ions, such that the spinel phase of the catalyst has the formula:

[(Cu²⁺ₓFe²⁺₁₋ₓ)(Fe³⁺_{y}Al³⁺_{1-y})₂O₄]

where x = 0.54 and y = 0.65.

### Example 2 (comparative)

### Preparation of a catalyst consisting of spinel phase only [(Cu²⁺ₓFe²⁺₁₋ₓ)(Fe³⁺_{y}Al³⁺_{1-y})₂O₄; (x=0.72, y=0.71)]

The catalyst was prepared according to procedure described in Example 1. However, in the present example the adjustment of pH value to 6.8 was achieved by adding 200 cm³ of aqueous NH₄OH solution with concentration of ammonium hydroxide of 10%. The fast basification caused the inclusion of more Cu and Fe ions in (Cu²⁺ₓFe²⁺₁₋ₓ)(Fe³⁺_{y}Al³⁺_{1-y})₂O₄ spinel phase with x = 0.72 and y = 0.71. Correspondingly, no Fe₂O₃ hematite phase was formed after calcination at 450°C. The material had the following weight ratio of metal components (EDAX): Fe:Al:K:Cu = 100:15:5:6, surface area 138 m²/gram, pore volume 0.20 cm³/gram and average pore diameter 4.3 nm.

### Example 3 (comparative)

### Preparation of a catalyst consisting of hematite phase [Fe₂O₃] and spinel phase of the formula [Fe²⁺(Fe³⁺_{y}Al³⁺_{1-y})₂O₄]; (y=0.40)]

The catalyst was prepared according to the procedure described in Example 1 but without any addition of Cu(NO₃)₂ salt to the mixed solution of metal nitrates. The material has following weight ratio of metal components (EDAX): Fe:Al:K = 100:15:6, surface area 107 m²/gram, pore volume 0.20 cm³/gram and average pore diameter 3.4 nm. The material contained two phases (XRD): 41 wt% of hematite Fe₂O₃ and 59 wt% of phase with spinel structure which composition reflects partial isomorphous substitution of Fe³⁺ ions with Al³⁺ ions, such that the spinel phase of the catalyst is represented by the following formula:

[Fe²⁺(Fe³⁺_{y}Al³⁺_{1-y})₂O₄]

where y= 0.40.

### Example 4 (reference)

### Preparation of a catalyst consisting of hematite phase [Fe₂O₃] and spinel phase of the formula [Fe²⁺(Fe³⁺_{y}Al³⁺_{1-y})₂O₄] (y=0.64)]

The catalyst was prepared according to the procedure described in Example 1, but without any addition of Cu(NO₃)₂·3H₂O salt to the mixed solution of metal nitrates. In this example 94 gram of Fe(NO₃)₃·9H₂O salt was used for preparation of iron salt solution in 50 cm³ water and for adjustment of the pH value to 6.8, 535 cm³ of 3% NH₄OH aqueous solution were added to the metal salt solution. The resultant material has the following weight ratio of metal components (EDAX): Fe:Al:K = 100:12:5, surface area 113 m²/gram, pore volume 0.21 cm³/gram and average pore diameter 4.9 nm. The material contained two phases (XRD): 45 wt% of hematite Fe₂O₃ and 55 wt% of phase with spinel structure whose composition reflects partial isomorphous substitution of Fe³⁺ ions with Al³⁺ ions, such that the spinel phase of the catalyst is represented by the formula:

[Fe²⁺(Fe³⁺_{y}Al³⁺_{1-y})₂O₄]

where y = 0.64.

### Example 5 (comparative)

### Preparation of a catalyst consisting of hematite phase [Fe₂O₃] and spinel phase of the formula [Fe²⁺(Fe³⁺_{y}Al³⁺_{1-y})₂O₄ (y=0.70)]

The catalyst was prepared according to the procedure described in Example 1 but without the addition of Cu(NO₃)₂·₃H₂O salt to the mixed solution of metal nitrates. In this example 115.7 gram of Fe(NO₃)₃·9H₂O salt was used for preparation of iron salt solution in 62 cm³ of water and for the adjustment of the pH value to 6.8 was added 570 cm³ of 3% NH₄OH aqueous solution. The material obtained has the following weight ratio of metal components (EDAX): Fe:Al:K = 100:9:4, surface area 93 m²/gram, pore volume 0.21 cm³/gram and average pore diameter 6.5 nm. The material contained two phases (XRD) : 86 wt% of hematite Fe₂O₃ and 14 wt% of phase with spinel structure whose composition reflects isomorphous substitution of Fe³⁺ ions with Al³⁺ ions, such that the spinel phase of the catalyst is represented by the formula [Fe²⁺(Fe³⁺_{y}Al³⁺_{1-y})₂O₄] where y = 0.70.

### Example 6 (comparative)

### Preparation of a catalyst consisting of iron oxide phases [Fe₂O₃; Fe₃O₄]

The procedure is according to US 5,140,049.

Iron Oxide catalyst was obtained by combustion synthesis using glycolic acid as organic complexant. 12.6 gram of Glycolic acid were dissolved in 10% aqueous ammonia up to pH 6.5 and slowly added to a solution containing 67 gram of Fe(NO₃)₃ · 9 H₂O dissolved in 50 cm³ of water. Then the water, ammonia and part of NO₃⁻ ions converted to NO/NO₂ were removed from the mixed solution in rotavapor increasing the temperature from 40 to 80°C up to complete drying of the material. The dried precipitate was calcined in air at 350°C for 1 h (heating rate 5°C min⁻¹). The formed iron oxide was impregnated by incipient wetness method with aqueous K₂CO₃ solution, followed by drying in air at 110°C overnight. The material consisted on 35 wt% Hematite Fe₂O₃ and 65 wt% Magnetite Fe₃O₄. It had surface area of 110 m²/gram, pore volume 0.31 cm³/gram and average pore diameter 4.3 nm.

### Example 7 (of the invention)

### Preparation of a catalyst consisting of copper-free spinel phase only [Fe(Fe³⁺_{y}Al³⁺_{1-y})₂O₄; (y=0.47)]

The catalyst was prepared by co-precipitation from the aqueous solution of Fe and Al nitrates by gradual addition of aqueous ammonium hydroxide solution. 27.0 gram of Al(NO₃)₃·9H₂O and 57.9 gram of Fe(NO₃)₃·9H₂O were dissolved in 60 cm³ of distilled water each. The solutions were then mixed together and the pH value was adjusted to 8.0 by gradually adding 250 cm³ of aqueous NH₄OH solution with concentration of ammonium hydroxide of 5 wt%. The addition time was 15 minutes. The precipitate was recovered and impregnated with K₂CO₃ solution as described in Example 1. In the present example the atomic ratio of Fe:Al in the precipitating solution was 2:1. Correspondingly, no Fe₂O₃ hematite phase was formed after calcination at 450°C. The material had the following weight ratio of metal components (EDAX): Fe:Al:K = 100:24:6, surface area 128 m²/gram, pore volume 0.53 cm³/gram and average pore diameter 3.5 nm.

### Example 8 (of the invention)

### Preparation of a catalyst consisting of copper-free spinel phase only [Fe(Fe³⁺_{y}Al³⁺_{1-y})₂O₄; (y=0.31)]

The catalyst was prepared by co-precipitation from the aqueous solution of Fe and Al nitrates by addition of aqueous ammonium hydroxide solution as described in Example 7. After mixing the aqueous solutions of Fe- and Al-salts the pH value was adjusted to 8.5 by gradually adding 265 cm³ of an aqueous NH₄OH solution (with concentration of 5 wt%). The addition time was 15 minutes. The precipitate was recovered and impregnated with K₂CO₃ solution as described in Example 1. The material consisted of only one - Al-substituted Fe₃O₄ spinel phase of formula Fe(Fe³⁺_{0.31}Al³⁺_{0.69})₂O₄ and had following weight ratio of metal components (EDAX): Fe:Al:K = 100:42:6, surface area 118 m²/gram, pore volume 0.43 cm³/gram and average pore diameter 3.0 nm.

### Example 9 (of the invention)

### Preparation of a catalyst consisting of copper-free spinel phase only [Fe(Fe³⁺_{y}Al³⁺_{1-y})₂O₄; (y=0.71)]

The catalyst was prepared by co-precipitation from the aqueous solution of Fe and Al nitrates by addition of aqueous ammonium hydroxide solution as described in Example 7. After mixing the aqueous solutions of Fe- and Al-salts the pH value was adjusted to 7.2 by gradually adding 100 cm³ of aqueous NH₄OH solution with concentration of ammonium hydroxide of 5 wt%. The addition time was 15 minutes and the precipitate was recovered and impregnated with K₂CO₃ solution as described in Example 1. The material consisted of only one - Al-substituted Fe₃O₄ spinel phase of formula Fe(Fe³⁺_{0.71}Al³⁺_{0.29})₂O₄ and had following weight ratio of metal components (EDAX): Fe:Al:K = 100:12:6, surface area 103 m²/gram, pore volume 0.38 cm³/gram and average pore diameter 3.7 nm.

### Example 10 (comparative)

### Preparation of a catalyst consisting on iron, copper and potassium deposited on Al₂O₃ support

(According to WO 97/05088, WO 01/34538 A1, US 4,555,526). To 36 gram of Fe(NO₃)₃·9H₂O, 1.25 gram of Cu(NO₃)₂.3H₂O and 3 gram of K₂CO3 dissolved in 100 cm³ of distilled water was added 20 gram of a powder of γ-Al2O3 with surface area of 180 m²/gram. The mixture was vigorously stirred and heated at 80°C to evaporate water. After evaporation of water, the solid reaction mixture was dried at 120°C in air for 24 hours and calcined at 450°C for 6 hours. The obtained catalyst had following weight ratio of metal components (EDAX): Fe:Cu:Al:K = 100:6:21:0.34, surface area 76 m²/gram, pore volume 0.16 cm³/gram and average pore diameter 8.2 nm.

The compositions of the catalysts of Examples 1 to 10 are tabulated in Table A.

**Table A**

| catalyst | Hematite phase Fe₂O₃ | Spinel phase (Cu²⁺ₓFe²⁺₁₋ₓ)(Fe³⁺_{y}Al³⁺_{1-y})₂O₄ |
|---|---|---|
| 1 comparative | 30% wt% | 70% wt% |
| | | x=0.54, y=0.65 |
| 2 comparative | 0% | 100 wt% |
| | | x=0.72, y=0.71 |
| 3 comparative | 41% (wt%) | 59% (wt%) |
| | | x=0.00, y=0.40 |
| 4 comparative | 45% (wt%) | 55% (wt%) |
| | | x=0.00, y=0.64 |
| 5 comparative | 86% (wt%) | 14% (wt%) |
| | | x=0.00, y=0.70 |
| 6 comparative | 20% (wt%) | |
| | Fe₃O₄ 80% (wt%) | |
| 7 of the invention | 0% | 100 wt% |
| | | x= 0.0; y = 0.47 |
| 8 of the invention | 0% | 100 wt% |
| | | x = 0.0; y = 0.31 |
| 9 of the invention | 0% | 100 wt% |
| | | x = 0.0; y = 0.71 |
| 10 comparative | Fe-Cu-K/Al2O3 | |

In the next set of examples (Examples 11-22), the catalysts of Examples 1-10 were tested for their activity and selectivity in CO₂ hydrogenation reaction to form fuel compositions. Catalysts testing included catalyst activation, reaction and products analysis.

Catalysts activation was done by means of two methods: reduction in hydrogen at 100 cm³/min^{∗}gram_{cat}, temperature 450°C, atmospheric pressure for 24 h; and carburization in a mixture of carbon monoxide and hydrogen in helium at flows 30:30:140 cm³/min^{∗}gram_{cat} respectively, at temperature 300°C, atmospheric pressure for 3 h.

CO₂ hydrogenation reaction was conducted by passing a mixture of H₂ and CO₂ flows at ratio 4:1 through the 3 gram of catalyst mixed with 1.8 gram of inert silica packed in a tubular reactor (11 mm ID and 210 mm long, 45 mm catalytic phase) and heated up to 300°C or 320°C at the total pressure of 10 atm. The reaction products were cooled down to +4°C and separated in cooled (+4°C) container. Gas products were analyzed in online Agilent 7890A Series Gas Chromatograph equipped with 7 columns and 5 automatic valves using helium as a carrier gas. Liquid products were separated into aqueous and organic phases. Aqueous phase was analyzed for Total Organic Carbon in Shimadzu TOC-V_{CPN} Analyzer. The liquid organic phase composition was analyzed by Agilent 190915-433 Gas Chromatograph combined with Mass Spectrometer in the range M/Z= 33-500, equipped with 5973 mass selective detector, HP-5MS column (30 m, 250 µm, i.d. 0.25 µm) and helium as a carrier gas. The distillation patterns of hydrocarbon oil were estimated by simulated distillation method based on the maximal boiling points of components in 10 vol% oil fractions. The oil productivity was calculated based on the weighted amounts of organic liquid (Wₒᵢₗ) collected over a specific period of time on stream: OP = Wₒᵢₗ /W_{cat}/RT gram/gram catalyst/h, where W_{cat} is weight of catalyst (gram) loaded to reactor, RT -time period on stream (hours). The selectivity to CO, CH₄, C₂-C₅ and C₆₊ hydrocarbons was calculated on the carbon basis as Sᵢ = [Cᵢ /(C_{CO2}^{∗}X_{CO2})] ^{∗} 100%, where Cᵢ amount of carbon (gram) contained in product (i) produced at period of time, C_{CO2} - amount of carbon (gram) contained in CO₂ passed the reactor at the same period of time, X_{CO2} - CO₂ conversion. In the tables below, the following abbreviations are used: Con for conversion, Sel for selectivity and Pro for productivity.

### Example 11 (reference)

### Carbon dioxide hydrogenation in one fixed bed reactor

The Catalyst of Example 1 was packed in a fixed bed reactor and activated by H₂ reduction as described above. The effect of WHSV on the reaction products was determined. The results are shown in Table 1. Increasing of the space time by decreasing of WHSV of CO₂ did not increase the CO₂ conversion beyond 60%.

**Table 1**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10² |
| 2 | 36 | 9 | 13 | 44 | 11.3 | 25 | 6.4 |
| 1 | 46 | 9 | 9 | 42 | 6.3 | 29 | 4.3 |
| 0.5 | 52 | 9 | 7 | 46 | 4.3 | 30 | 2.8 |
| 0.2 | 59 | 9 | 5 | 44 | 1.9 | 35 | 1.5 |
| 0.1 | 60 | 9 | 4 | 35 | 0.7 | 29 | 0.6 |

### Example 12 (reference)

### Carbon dioxide hydrogenation in one fixed bed reactor

The catalyst of Example 1 was packed in a fixed bed reactor and activated by carburization as described above. The effect of WHSV upon the reaction products was determined. The results are shown in Table 2. Comparison of the results presented in Tables 1 and 2 indicates a significantly higher activity of the catalyst after carburization compared with its activation by hydrogen reduction. Higher CO₂ conversions and oil productivities were measured at different WHSV with carburized catalyst.

**Table 2**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10² |
| 6 | 40 | 10 | 12 | 38 | 32.2 | 29 | 24.6 |
| 3 | 46 | 11 | 7 | 41 | 20.1 | 26 | 12.5 |
| 1 | 53 | 8 | 5 | 15 | 10.9 | 19 | 3.5 |

Reaction conditions: Temperature 300°C, Total pressure 10 atm, H₂/CO₂ = 4 mol/mol.

### Example 13 (reference)

### Carbon dioxide hydrogenation in one fixed bed reactor

Catalyst prepared as described in Example 3 was packed in a fixed bed reactor and activated by carburization as described above. The effects of WHSV on the reaction products were determined. The results are shown in Table 3.

**Table 3**

| CO₂ WHSV h⁻¹ | CO₂ Con**.**, % | Methane Sel. % | CO Sel. % | C₂-C₅ | | C₆₊ oil | |
|---|---|---|---|---|---|---|---|
| | | | | Sel. % | Pro. (g/g_{Cat}•h)•10² | Sel. % | Pro. (g/g_{cat}•h)•10² |
| 1 | 52 | 9 | 10 | 45 | 8.3 | 25 | 4.7 |
| 6 | 41 | 10 | 20 | 44 | 36.3 | 15 | 12.3 |

Reaction conditions: Temperature 300°C, Total pressure 10 atm, H₂/CO₂=4.

### Example 14 (reference)

### Carbon dioxide hydrogenation in one fixed bed reactor

Catalyst prepared as described in Example 4 was packed in a fixed bed reactor and activated by carburization as described above. Effects of WHSV upon the reaction products were determined. The results are shown in Table 4.

**Table 4**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10² |
| 1 | 53 | 9 | 6 | 46 | 8.5 | 24 | 4.5 |
| 6 | 33 | 6 | 31 | 41 | 27.4 | 15 | 9.8 |

Reaction conditions: T = 300°C, P=10 atm, H₂/CO₂=4.

### Example 15 (reference)

### Carbon dioxide hydrogenation in one fixed bed reactor

Catalyst prepared as described in Example 5 was packed in a fixed bed reactor and activated by carburization as described above. The effects of WHSV on the reaction products were determined. The results are shown in Table 5.

**Table 5**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10² |
| 1 | 37 | 8 | 14 | 50 | 6.6 | 20 | 3.5 |
| 6 | 20 | 7 | 83 | 1 | 0.2 | 3 | 1.0 |

Reaction conditions: T=300°C, P=10 atm, H₂/CO₂=4.

### Example 16

### Carbon dioxide hydrogenation in one fixed bed reactor in the presence of a catalyst consisting of spinel phase only (Cu-free)

Catalyst prepared as described in Example 7 was packed in a fixed bed reactor and activated by carburization as described above. The effect of WHSV upon the reaction products was determined. The results are shown in Table 6.

**Table 6**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10² |
| 1 | 52 | 12 | 6 | 39 | 7.9 | 29 | 5.8 |
| 6 | 45 | 12 | 13 | 27 | 26 | 40 | 38.5 |

Reaction conditions: T = 300°C, Total pressure 10 atm, H₂/CO₂=4.

The selectivities to C₆₊ hydrocarbons and oil productivity obtained with this catalyst of the present invention are significantly higher compared to that measured with carburized K/FeOₓ or biphasic K/Cu-Fe-Al-O-Fe₂O₃, K/Fe-Al-O-Fe₂O₃ catalysts.

### Example 17 (reference)

### Carbon dioxide hydrogenation in one fixed bed reactor in the presence of a catalyst consisting of spinel phase only (containing Cu)

Catalyst prepared as described in Example 2 was packed in a fixed bed reactor and activated by H₂. The effect of WHSV upon the reaction products was determined. The results are shown in Table 7.

**Table 7**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10 2 |
| 1 | 35 | 9 | 28 | 39 | 4.9 | 16 | 2.0 |
| 6 | 23 | 8 | 39 | 46 | 22.6 | 6 | 2.9 |

Reaction conditions: T = 300°C, Total pressure 10 atm, H₂/CO₂=4.

### Example 18 (reference)

### Testing in one fixed bed reactor of a catalyst consisting of iron oxide phases [Fe₂O₃; Fe₃O₄] and not containing spinel phase

The catalyst prepared according to Example 6 was packed in the tubular reactor and activated by carburization as described above. The testing results are shown in Table 8.

**Table 8**

| CO₂ Con., % | Methane Sel. % | CO Sel. % | C₂-C₅ | | C₆₊ oil | |
|---|---|---|---|---|---|---|
| | | | Sel. % | Pro. (g/g_{cat}•h)•10² | Sel. % | Pro. (g/g_{cat}•h)•10² |
| 39 | 8 | 16 | 46 | 5.9 | 21 | 2.7 |

Reaction conditions: 300°C, 10 atm, H₂/CO₂=4, WHSV=1.0 h⁻¹

### Example 19 (reference)

### Carbon dioxide hydrogenation in one fixed bed reactor

The catalyst prepared according to Example 9 was packed in the tubular reactor and activated by hydrogen reduction as described above. The testing results are shown in Table 9.

**Table 9**

| CO₂ Con. , % | Methane Sel. % | CO Sel. % | C₂-C₅ | | C₆₊ oil | |
|---|---|---|---|---|---|---|
| | | | Sel. % | Pro. (g/g_{cat}•h)•10² | Sel. % | Pro. (g/g_{cat}•h)•10² |
| 27 | 10 | 28 | 48 | 4.6 | 11 | 1.1 |

Reaction conditions: Temperature 300°C, total pressure 10 atm, H₂/CO₂=4, WHSV= 1 h⁻¹.

### Example 20

### Carbon dioxide hydrogenation in one fixed bed reactor

The catalyst prepared according to Example 8 was packed in the tubular reactor and activated by carburization as described above. The testing results are shown in Table 10.

**Table 10**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10² |
| 1 | 52 | 13 | 6 | 41 | 7.6 | 27 | 5.0 |

Reaction conditions: Temperature 300°C, total pressure 10 atm, H₂/CO₂=4, WHSV= 1 h⁻¹.

### Example 21

### Carbon dioxide hydrogenation in one fixed bed reactor

The catalyst prepared according to Example 9 was packed in the tubular reactor and activated by carburization as described above. The testing results are shown in Table 11.

**Table 11**

| CO₂ WHSV h⁻¹ | CO₂ Con., % | Methane Sel. % | CO Sel. % | C₂-C₅ | | C₆₊ oil | |
|---|---|---|---|---|---|---|---|
| | | | | Sel. % | Pro. (g/g_{cat}•h)•10² | Sel. % | Pro. (g/g_{cat}•h)•10² |
| 1 | 53 | 13 | 7 | 40 | 7.6 | 28 | 5.3 |

Reaction conditions: Temperature 300°C, total pressure 10 atm, H₂/CO₂=4, WHSV= 1 h⁻¹.

### Example 22 (reference)

### Carbon dioxide hydrogenation in one fixed bed reactor

The catalyst prepared according to Example 10 was packed in the tubular reactor and activated by hydrogen reduction as described above. The testing results are shown in Table 12.

**Table 12**

| CO₂ Con., % | Methane Sel. % | CO Sel. % | C₂-C₅ | | C₆₊ oil | |
|---|---|---|---|---|---|---|
| | | | Sel. % | Pro. (g/g_{cat}•h)•10² | Sel. % | Pro. (g/g_{cat}•h)•10² |
| 38 | 8 | 15 | 58 | 7.9 | 8.6 | 1.2 |

Reaction conditions: Temperature 300°C, total pressure 10 atm, H₂/CO₂=4, WHSV= 1 h⁻¹.

CO₂ conversion levels achieved by the catalysts of Examples 1-10, when operating under the following set of experimental conditions: Temperature 300°C, total pressure 10 atm, H₂/CO₂=4, WHSV_{CO2}= 1 h⁻¹, as reported in Examples 11-22, are tabulated in Table B.

**Table B**

| catalyst | CO₂ conversion % |
|---|---|
| 1 | 53 |
| 2 | 35 |
| 3 | 52 |
| 4 | 53 |
| 5 | 37 |
| 6 | 39 |
| 7 | 52 |
| 8 | 52 |
| 9 | 53 |
| 10 | 40 |

In the next set of examples (Examples 23-27), some of the catalysts demonstrating CO₂ conversion of slightly more than 50% according to the data tabulated in Table B (i.e., the catalysts of Examples 1, 3, 4 and 7) were tested in an experimental set-up consisting of three reactors positioned in series, with water removal taking place between each pair of consecutive reactors, i.e., between the first and second reactors, and between the second and third reactors.

### Example 23 (reference)

### Carbon dioxide hydrogenation in three reactors in series

6 gram of the catalyst prepared as described in Example 1 was packed in three fixed bed reactors in series. 2 gram of catalyst was packed in each reactor and activated by carburization as described above. The testing system consisted of 3 consecutive SS fixed bed reactors with pressure, temperature and flow controls (shown in Figure 4). After each reactor the heavy organic products (oil) and water were condensed in a cooling system and collected in the tank. After separation of liquid products, the gases containing non-reacted CO₂, H₂ and light Hydrocarbons, C₁-C₅ proceeded to flow to the next reactor. After each reactor the gas and liquid products were analyzed as described above. All the three reactors were kept at the same temperature and total pressure of 10 atm.

The catalyst performance as a function of temperature, H₂:CO₂ molar ratio and the number of reactors (N) in the testing system is listed in Table 13.

**Table 13**

| N | T °C | H₂:CO₂ | CO₂ WHSV h⁻¹ | CO₂ Con. % | CH₄ Sel. % | CO Sel. % | C₂-C₅ | | C₆₊ oil | |
|---|---|---|---|---|---|---|---|---|---|---|
| | | | | | | | Sel. % | Pro. (g/g_{cat}•h)•10² | Sel. % | Pro. (g/g_{cat}•h)•10² |
| 1 | 300 | 4:1 | 6 | 40 | 12 | 11 | 35 | 26.6 | 31 | 23.2 |
| 2 | 300 | 4:1 | 3 | 62 | 12 | 5 | 40 | 23.4 | 33 | 19.3 |
| 3 | 300 | 4:1 | 2 | 77 | 12 | 2 | 42 | 34.2 | 34 | 17.6 |
| 3 | 320 | 3:1 | 2 | 72 | 9 | 3 | 32 | 37.8 | 38 | 18.1 |
| 3 | 320 | 4:1 | 2 | 90 | 12 | 1 | 44 | 34.0 | 34 | 19.0 |
| 3 | 320 | 6:1 | 2 | 93 | 18 | 1 | 47 | 26.7 | 27 | 16.2 |

### Example 24 (reference)

### Carbon dioxide hydrogenation in three reactors in series

6 gram of catalyst prepared as described in Example 3 was packed in three fixed bed reactors in series as set out in Example 23. The aqueous and liquid hydrocarbons phases were separated from light gases between first and second and between second and third reactors as shown in Figure 4. The catalyst in all three reactors was activated by carburization as described above and tested as in Example 23. The effects of temperature and total pressure upon the reaction products were determined. The results are shown in Table 14. The oil hydrocarbon composition was similar to that obtained for oil collected in Example 23.

**Table 14**

| N | T, °C | P, atm | CO₂, WHSV h⁻¹ | CO₂ Con. % | CH₄ Sel. % | CO Sel. % | C₂-C₅ | | C₆₊ oil | |
|---|---|---|---|---|---|---|---|---|---|---|
| | | | | | | | Sel. % | Pro. (g/g_{cat}•h) •10² | Sel. % | Pro. (g /g_{cat}•h)•10² |
| 1 | 300 | 10 | 6 | 41 | 10 | 20 | 44 | 36.3 | 15 | 12.3 |
| 3 | 300 | 10 | 2 | 79 | 10 | 4 | 42 | 21.8 | 33 | 17 |
| 3 | 320 | 10 | 2 | 84 | 11 | 2 | 38 | 21.2 | 37 | 20.7 |
| 3 | 320 | 15 | 2 | 86 | 12 | 2 | 30 | 16.6 | 43 | 24.0 |
| 3 | 320 | 5 | 2 | 62 | 9 | 30 | 40 | 16.7 | 16 | 6.9 |

Reaction conditions: H₂/CO₂=4.

### Example 25 (reference)

### Carbon dioxide hydrogenation in three reactors in series

6 gram of catalyst prepared as described in Example 6 was packed in three fixed bed reactors in series as set out in Example 23. The catalyst was activated by carburization as described above and tested as in Example 23. The effect of WHSV upon the reaction products was determined. The results are shown in Table 15. The oil hydrocarbon composition was similar to that obtained for oil collected in Example 23.

**Table 15**

| N | CO₂, WHSV h⁻¹ | CO₂ Con. % | CH₄ Sel. % | CO Sel. % | C₂-C₅ | | C₆₊ oil | |
|---|---|---|---|---|---|---|---|---|
| | | | | | Sel. % | Pro. (g/g_{cat}•h)•10² | Sel. % | Pro. (g/g_{cat}•h)•10² |
| 3 | 1 | 70 | 10 | 10 | 34 | 7.8 | 28 | 6.5 |
| 3 | 2 | 57 | 8 | 20 | 39 | 14.0 | 26 | 10.6 |

Reaction conditions: T= 300°C, H₂/CO₂=4, total pressure 10 atm.

### Example 26

### Carbon dioxide hydrogenation in three reactors in series in the presence of a catalyst consisting of spinel phase only (Cu-free catalyst)

6 gram of catalyst prepared as described in Example 7 was packed in three fixed bed reactors in series as set out in Example 23. The aqueous and liquid hydrocarbons phases were separated from light gases between first and second and between second and third reactors as shown in the scheme of Figure 4. The catalyst in all three reactors was activated by carburization as described above and tested as in Example 23. The effects of WHSV_{CO2} upon the reaction products were determined. The results are shown in Table 16. The cascade reactors configuration with this catalyst of the present invention gives maximal Cₑ+ hydrocarbons selectivity and oil productivity about 0.5 kg/kg_{cat} h.

**Table 16**

| N | T, °C | P, atm | CO₂, WHSV h⁻¹ | CO₂ Con. % | CH₄ Sel. % | CO Sel. % | C₂-C₅ | | C₆₊ oil | |
|---|---|---|---|---|---|---|---|---|---|---|
| | | | | | | | Sel. % | Pro. (g/g_{cat}•h)•10² | Sel. % | Pro. (g/g_{cat}•h)•10² |
| 1 | 320 | 10 | 2 | 54 | 10 | 11 | 36 | 12.3 | 32 | 10.9 |
| 3 | 320 | 10 | 2 | 89 | 13 | 4 | 30 | 18.8 | 47 | 29.6 |
| 3 | 320 | 10 | 6 | 81 | 15 | 7 | 35 | 44.9 | 38 | 59.2 |

Reaction conditions: T = 320°C, Total pressure 10 atm, H₂/CO₂=4.

### Example 27

### Carbon dioxide/Carbon monoxide hydrogenation in three reactors in series

6 gram of catalyst prepared as described in Example 7 was packed in three fixed bed reactors in series with pressure, temperature and flow controls as set out in Example 23. 2 gram of the catalyst was packed in each reactor and activated by carburization as described above. The catalyst was tested in hydrogenation reaction of the mixture of CO₂ and CO by feeding a mixture of H₂, CO₂ and CO flows at molar ratio 5:1:1, respectively. After each reactor the heavy organic products (oil) and water were condensed in a cooling system and collected in the tank. After separation of liquid products, the gases containing non-reacted CO₂, CO and H₂ and light hydrocarbons flowed to the next reactor. The gas products were analyzed as described above after the third reactor. The liquids were collected from three reactors were mixed and analyzed as described above. All the three reactors were kept at the same temperature and total pressure. The oil productivity was calculated based on the weighted amounts of organic liquid (Wₒᵢₗ) collected per hour: OP = Wₒᵢₗ/W_{cat}/h, where W_{cat} is total weight of loaded catalyst. The selectivity to CH₄, C₂-C₅ and C₆+ hydrocarbons was calculated on the carbon basis as Sᵢ = [Cᵢ /(C_{CO2+CO}^{∗}X_{Total})] ^{∗} 100%, where Cᵢ amount of carbon (gram) contained in product (i) produced at period of time, C_{CO2+CO} - amount of carbon (gram) contained in both CO₂ and CO that flowed through the reactor at the same period of time, X_{Total} - total conversion of C calculated as C_{reacted}/Cᵢₙ (weight fraction). The effect of pressure upon the reaction products was determined. The results are shown in Table 17.

**Table 17**

| CO₂ WHSV h⁻¹ | CO WHSV h⁻¹ | CO₂+CO WHSV h⁻¹ | P atm | CO₂ Con % | CO Con % | CH₄ Sel % | Total Con% | C₂-C₅ | | C₆₊ oil | |
|---|---|---|---|---|---|---|---|---|---|---|---|
| | | | | | | | | Sel % | Pro. (g/g_{cat}•h) •10² | Sel % | Pro. (g/g_{cat}•h) •10² |
| 1.0 | 0.6 | 1.6 | 10 | 55 | 97 | 13.4 | 73.2 | 52.4 | 22.1 | 29 | 12.3 |
| 1.0 | 0.6 | 1.6 | 15 | 66 | 98 | 14.1 | 80.0 | 50 | 23.1 | 30 | 13.9 |

Reaction conditions: T= 320°C, H₂/CO₂/CO=5:1:1.

### Example 28

### Carbon dioxide hydrogenation in three reactors in series with different catalysts at CO₂ conversion of 75% adjusted by varying of WHSV_{CO2}

In the set of experiments described in this Example, the performance (activity and selectivity) of the catalysts tabulated in Table B was measured under identical conditions (the same feed composition, temperature, pressure and CO₂ conversion). The latter variable is controlled by varying the space velocity. Using the three packed-bed-reactors-in-series system illustrated in Figure 4, high CO₂ conversion (75%) was reached to reflect the selectivity to hydrocarbons.

Thus, 6 gram of catalysts prepared as described in Examples 1, 3, 4, 6, 7, 8 and 9 were packed in separate experiments in three fixed bed reactors in series as set out in Example 23. The catalysts were activated by carburization as described above. The results set out in Table 18 were measured for every catalyst after 200 h of run at 300°C, P = 10 atm, H₂/CO₂ = 4 and properly adjusted WHSV_{CO2} to produce total CO₂ conversion of 75%.

**Table 18**

| Catalyst | WHSV_{CO2} h⁻¹ | Products selectivity, wt% | | | | | | C5+ pro. g/g_{cat}•h•10⁻² |
|---|---|---|---|---|---|---|---|---|
| | | CH₄ | CO | C2-C4 Olefins | C2-C4 paraffins | C2-C4 Oxygenates in water | C5+ | |
| 1 | 2.0 | 12 | 2 | 29 | 8 | 7 | 41 | 20.3 |
| 3 | 1.5 | 10 | 3 | 30 | 10 | 9 | 38 | 15.2 |
| 4 | 1.5 | 11 | 4 | 29 | 9 | 8 | 37 | 14.8 |
| 6 | 1.0 | 10 | 9 | 29 | 8 | 8 | 35 | 8.2 |
| 7 | 6.0 | 16 | 3 | 28 | 8 | 7 | 38 | 54.8 |
| 8 | 5.0 | 14 | 3 | 29 | 9 | 7 | 38 | 50.8 |
| 9 | 4.0 | 15 | 4 | 28 | 8 | 8 | 37 | 40.6 |

The results shown in Table 18 demonstrate that the catalysts consisting of pure phase of Al-substituted Fe₃O₄ with spinel structure, i.e., the catalysts represented by Formula 1 Fe²⁺(Fe³⁺_{y}Al³⁺_{1-y})₂O₄; (y=0.05 - 0.95, preferably y=0.25 - 0.75 and more preferably y=0.30 - 0.70), i.e., catalysts of Examples 7, 8 and 9, display higher catalytic activity in conversion of CO₂ to hydrocarbons compared with the catalysts of Examples 1, 3, 4 and 6.

### Example 29

### Preparation of extruded catalyst consisting of spinel phase only [Fe(Fe³⁺_{y}Al³⁺_{1-y})₂O₄; (x=0.00, y=0.47)]

Fe(Fe³⁺_{y}Al³⁺_{1-y})₂O₄ from Example 7 was formed as extrudates with cylindrical shape using Al₂O₃ as a binder. 11.73 gram of Fe(Fe³⁺_{y}Al³⁺_{1-y})₂O₄ prepared in Example 7 was mixed with 4.89 gram of AlOOH powder (Disperal P2, Sasol Ltd., Germany) and water was added to get a slurry. The slurry was kneeded in a mortar and extruded using a matrix with 1.2 mm cylindrical shape opening. The extrudates were dried at room temperature for 24 h, then at 110°C overnight and calcined at 450°C for period of 2 h (temperature increasing rate 5°C/min). The so-formed material was impregnated by incipient wetness with aqueous solution containing 2.15 gram K₂CO₃. The impregnated material was dried in air at 110°C for period of 24 h and calcined in air at 450°C for period of 2 h (temperature increasing rate 5°C/min).

## Claims

1. A catalyst suitable for use in the hydrogenation of carbon dioxide-containing gas, said catalyst consisting of potassium promoted spinel phase of the following formula:
K/[Fe²⁺(Fe³⁺_{y}Al³⁺_{1-y})₂O₄] Formula
wherein y is from 0.25 to 0.75,
wherein the catalyst exhibits surface area of not less than 80 m²/gram by the BET method, and optionally a binder, and
wherein the potassium is applied onto the surface of said spinel.

2. A catalyst according to claim 1, wherein y is from 0.3 to 0.7.

3. A catalyst according to claim 1 or 2, wherein the spinel of the Formula has average crystal size in the range from 1.5 to 3 nm, as measured by X-Ray diffraction (XRD).

4. A catalyst according to any of claims 1 to 3, wherein the catalyst exhibits surface area between 80 and 180 m²/gram.

5. A process for preparing a catalyst of Formula 1:
[Fe²⁺(Fe³⁺_{y}Al³⁺_{1-y})₂O₄] Formula 1
wherein y is in the range from 0.25 to 0.75; comprising dissolving in an aqueous solution at least one ferric compound and at least one aluminum compound, wherein the weight ratio Al:Fe in the metal-containing solution is not less than 20:100, gradually adjusting the pH of the metal-containing solution by means of addition of ammonium hydroxide, whereby co-precipitation of the metals in the form of their hydroxides occurs, wherein the adjustment of the pH of the aqueous solution is to the range from 7.0 to 8.5, separating the solid formed from the liquid phase, and subjecting same to drying and calcinations in air at a temperature in the range from 400 to 500°C for at least 6 hours to obtain the hematite-free spinel of Formula 1, wherein the catalyst exhibits surface area of not less than 80 m²/gram by the BET method.

6. A process according to claim 5, wherein the pH is adjusted within the range from 7.5 to 8.0, wherein the addition time of the ammonium hydroxide is not less than 10 minutes.

7. A process according to claim 5 or 6, wherein a dilute aqueous solution of ammonium hydroxide is added, with concentration of not more than 5% by weight.

8. A process according to claim 6 or 7, wherein the metal-containing solution and the ammonium hydroxide solution are held at room temperature during the addition.

9. A process according to any one of claims 5 to 8, further comprising treating the dried material with an aqueous solution of potassium salt before the calcination step.

10. A process for the preparation of hydrocarbons comprising the hydrogenation of carbon dioxide-containing gas in the presence of the catalyst as defined in any of claims 1 to 4, wherein the catalyst is activated *in-situ* by means of carburization, said carburization comprises treating said catalyst with a mixture of H₂ and CO at elevated temperatures.

11. A process according to claim 10, employing a plurality of serially arranged reactors, comprising:
(i) feeding carbon dioxide-containing gas and hydrogen streams into at least the first of said serially arranged reactors which are loaded with the catalyst as defined in any of claims 1 to 4, wherein said catalyst was brought to an active form by means of carburization;
(ii) discharging a gaseous reaction stream from at least one of said serially arranged reactors and cooling same, to form a gas-liquid mixture, wherein the liquid component of said mixture comprises condensable reaction products consisting of water and organic liquids, and the gas component of said mixture comprises reactants and non-condensable reaction products;
(iii) separating said gaseous component from said liquid component;
(iv) separating said liquid component into an organic and aqueous phases, and collecting at least said organic phase; and
(v) feeding the gaseous component into the successive reactor in said serially arranged reactors.

## Patentansprüche

1. Ein Katalysator, geeignet zur Verwendung bei der Hydrierung von kohlendioxidhaltigem Gas, wobei der Katalysator aus einer Spinell-Phase mit Kaliumpromoter der folgenden Formel besteht:
K/[Fe²⁺(Fe³⁺_{y}Al³⁺_{1-y})₂O₄] Formel
wobei y 0,25 bis 0,75 beträgt,
wobei der Katalysator eine Oberfläche von nicht weniger als 80 m²/Gramm mit dem BET-Verfahren aufweist, und gebebenenfalls ein Bindemittel und
wobei das Kalium auf der Oberfläche des Spinells aufgebracht ist.

2. Ein Katalysator gemäß Anspruch 1, wobei y 0,3 bis 0,7 beträgt.

3. Ein Katalysator gemäß Anspruch 1 oder 2, wobei das Spinell der Formel eine durchschnittliche Kristallgröße im Bereich von 1,5 bis 3 nm aufweist, gemessen durch Röntgenstrahlbeugung (XRD).

4. Ein Katalysator gemäß einem der Ansprüche 1 bis 3, wobei der Katalysator eine Oberfläche zwischen 80 und 180 m²/Gramm aufweist.

5. Ein Verfahren zur Herstellung eines Katalysator der Formel 1:
[Fe²⁺(Fe³⁺_{y}Al³⁺_{1-y})₂O_{4]} Formel 1
wobei y im Bereich von 0,25 bis 0,75 liegt; umfassend das Lösen in einer wässrigen Lösung von mindestens einer Eisenverbindung und mindestens einer Aluminiumverbindung, wobei das Gewichtsverhältnis Al:Fe in der metallhaltigen Lösung nicht weniger als 20:100 beträgt, schrittweises Einstellen des pH-Wertes der metallhaltigen Lösung mittels Zugabe von Ammoniumhydroxid, wodurch Co-Ausfällung der Metalle in Form ihrer Hydroxide erfolgt, wobei das Einstellen des pH-Wertes der wässrigen Lösung auf den Bereich von 7,0 bis 8,5 stattfindet, Abtrennen des aus der Flüssigphase gebildeten Feststoffes und Unterziehen desselben Trocknen und Kalzinieren an der Luft bei einer Temperatur im Bereich von 400 bis 500°C für mindestens 6 Stunden, um das hematitfreie Spinell der Formel 1 zu erhalten, wobei der Katalysator eine Oberfläche von nicht weniger als 80 m²/Gramm mit einem BET-Verfahren aufweist.

6. Ein Verfahren gemäß Anspruch 5, wobei der pH-Wert innerhalb des Bereichs von 7,5 bis 8,0 eingestellt ist, wobei die Zugabezeit des Ammoniumhydroxids nicht weniger als 10 Minuten beträgt.

7. Ein Verfahren gemäß Anspruch 5 oder 6, wobei eine verdünnte wässrige Lösung von Ammoniumhydroxid mit einer Konzentration von nicht mehr als 5 Gew.-% zugegeben wird.

8. Ein Verfahren gemäß Anspruch 6 oder 7, wobei die metallhaltige Lösung und die Ammoniumhydroxidlösung während der Zugabe bei Raumtemperatur gehalten werden.

9. Ein Verfahren gemäß einem der Ansprüche 5 bis 8, ferner umfassend das Behandeln des getrockneten Materials mit einer wässrigen Lösung des Kaliumsalzes vor dem Kalzinierungsschritt.

10. Ein Verfahren zur Herstellung von Kohlenwasserstoffen, umfassend die Hydrierung von kohlendioxidhaltigem Gas in Gegenwart des Katalysators wie in einem der Ansprüche 1 bis 4 definiert, wobei der Katalysator *in-situ* mittels Aufkohlung aktiviert wird, wobei die Aufkohlung das Behandeln des Katalysators mit einem Gemisch aus H₂ und CO bei erhöhten Temperaturen umfasst.

11. Ein Verfahren gemäß Anspruch 10 unter Verwendung einer Vielzahl an in Reihe angeordneten Reaktoren, umfassend:
(i) Einspeisen von kohlendioxidhaltigem Gas und Wasserstoffströmen in mindestens den ersten der in Reihe angeordneten Reaktoren, welche mit dem Katalysator wie in einem der Ansprüche 1 bis 4 definiert beschickt werden, wobei der Katalysator mittels Aufkohlung in eine aktive Form gebracht wurde;
(ii) Abgabe eines gasförmigen Reaktionsstroms aus mindestens einem der in Reihe angeordneten Reaktoren und Kühlen derselben, um ein Gas-Flüssigkeitsgemisch zu bilden, wobei die flüssige Komponente des Gemischs kondensierbare Reaktionsprodukte bestehend aus Wasser und organischen Flüssigkeiten umfasst und die Gaskomponente des Gemischs Reaktanten und nicht-kondensierbare Reaktionsprodukte umfasst;
(iii) Abtrennen der gasförmigen Komponente aus der flüssigen Komponente;
(iv) Abtrennen der flüssigen Komponente in eine organische und wässrige Phasen und Sammeln mindestens der organischen Phase; und
(v) Einspeisen der gasförmigen Komponente in den nachfolgenden Reaktor der in Reihe angeordneten Reaktoren.

## Revendications

1. Catalyseur utilisable pour l'hydrogénation de gaz contenant du dioxyde de carbone, ledit catalyseur étant constitué d'une phase de spinelle favorisée par le potassium, de formule suivante :
K/[Fe²⁺(Fe³⁺_{y}Al³⁺_{1-y})₂O₄] Formule
dans laquelle y vaut de 0,25 à 0,75,
lequel catalyseur présente une surface spécifique non inférieure à 80 m²/gramme par la méthode BET,
et éventuellement un liant, et
dans lequel le potassium est appliqué sur la surface dudit spinelle.

2. Catalyseur selon la revendication 1, dans lequel y vaut de 0,3 à 0,7.

3. Catalyseur selon la revendication 1 ou 2, dans lequel le spinelle de la formule a une taille de cristal moyenne située dans la plage allant de 1,5 à 3 nm, telle que mesurée par diffraction des rayons X (XRD).

4. Catalyseur selon l'une quelconque des revendications 1 à 3, lequel catalyseur présente une surface spécifique comprise entre 80 et 180 m²/gramme.

5. Procédé pour préparer un catalyseur de formule 1 :
[Fe²⁺(Fe³⁺_{y}Al³⁺_{1-y})₂O₄] Formule 1
dans laquelle y est situé dans la plage allant de 0,25 à 0,75 ;
comprenant la dissolution, dans une solution aqueuse, d'au moins un composé ferrique et d'au moins un composé de l'aluminium, le rapport en poids Al/Fe dans la solution contenant du métal n'étant pas inférieur à 20/100, l'ajustement progressif du pH de la solution contenant du métal par addition d'hydroxyde d'ammonium, en conséquence de quoi il se produit une co-précipitation des métaux sous la forme de leurs hydroxydes, l'ajustement du pH de la solution aqueuse étant jusqu'à la plage allant de 7,0 à 8,5, la séparation du solide formé d'avec la phase liquide, et sa soumission à un séchage et à des calcinations dans l'air à une température située dans la plage allant de 400 à 500°C pendant au moins 6 heures pour que soit obtenu le spinelle sans hématite de formule 1, dans lequel le catalyseur présente une surface spécifique non inférieure à 80 m²/gramme par la méthode BET.

6. Procédé selon la revendication 5, dans lequel le pH est ajusté dans la plage allant de 7,5 à 8,0, et dans lequel le temps d'addition de l'hydroxyde d'ammonium n'est pas inférieur à 10 minutes.

7. Procédé selon la revendication 5 ou 6, dans lequel une solution aqueuse diluée d'hydroxyde d'ammonium est ajoutée, à une concentration non supérieure à 5 % en poids.

8. Procédé selon la revendication 6 ou 7, dans lequel la solution contenant du métal et la solution d'hydroxyde d'ammonium sont maintenues à la température ambiante durant l'addition.

9. Procédé selon l'une quelconque des revendications 5 à 8, comprenant en outre le traitement du matériau séché avec une solution aqueuse de sel de potassium avant l'étape de calcination.

10. Procédé pour la préparation d'hydrocarbures, comprenant l'hydrogénation de gaz contenant du dioxyde de carbone en présence du catalyseur tel que défini dans l'une quelconque des revendications 1 à 4, dans lequel le catalyseur est activé *in situ* au moyen d'une carburation, ladite carburation comprenant le traitement dudit catalyseur avec un mélange de H₂ et de CO à des températures élevées.

11. Procédé selon la revendication 10, employant une pluralité de réacteurs disposés en série, comprenant :
(i) l'introduction de courants d'hydrogène et de gaz contenant du dioxyde de carbone dans au moins le premier desdits réacteurs disposés en série qui sont chargés avec le catalyseur tel que défini dans l'une quelconque des revendications 1 à 4, lequel catalyseur a été mis sous une forme active au moyen d'une carburation ;
(ii) la décharge d'un courant réactionnel gazeux hors d'au moins un desdits réacteurs disposés en série et son refroidissement, pour former un mélange de gaz-liquide, dont le composant liquide dudit mélange comprend des produits réactionnels condensables constitués d'eau et de liquides organiques, et le composant gazeux dudit mélange comprend des réactifs et des produits réactionnels non condensables ;
(iii) la séparation dudit composant gazeux d'avec ledit composant liquide ;
(iv) la séparation dudit composant liquide en une phase organique et une phase aqueuse, et la collecte au moins de ladite phase organique ; et
(v) l'introduction du composant gazeux dans le réacteur successif parmi lesdits réacteurs disposés en série.
